# EUROPEAN PATENT SPECIFICATION

(11) **EP 3 394 648 B1**
(45) Date of publication and mention of the grant of the patent: **17.07.2019**
(21) Application number: 17709164.2
(22) Date of filing: 22.02.2017
(51) Int. Cl.: G02B 1/04, C11D 1/66

(54) **SILICONE HYDROGEL CONTACT LENSES HAVING IMPROVED LUBRICITY**
SILIKONHYDROGELKONTAKTLINSEN MIT HYDROPHILER SCHMIERFÄHIGKEIT
LENTILLES DE CONTACT EN SILICONE-HYDROGEL PRÉSENTANT UN POUVOIR LUBRIFIANT AMÉLIORÉ

(30) Priority: 22.02.2016 US 201662297957 P
(43) Date of publication of application: 31.10.2018
(73) Proprietor: CooperVision International Holding Company, LP, St. Michael (BB)
(72) Inventor: ROGERS, Victoria, Pleasanton, California 94588 (US); GEORGE, Melanie, Pleasanton, California 94588 (US); LUK, Andrew, Pleasanton, CA 94588 (US)
(74) Representative: Abel & Imray
(86) International application number: PCT/GB2017/050455
(87) International publication number: WO 2017/144874

(56) References cited:
- WO-A1-02/087326
- WO-A2-98/55155
- US-A1- 2003 130 144

## Description

### FIELD

The field of the invention relates to silicone hydrogel contact lenses, packages containing silicone hydrogel contact lenses, and methods of manufacturing silicone hydrogel contact lenses.

### BACKGROUND

Contact lenses made from silicone hydrogel materials are rapidly gaining popularity over contact lenses made from other materials because they are comfortable to wear and, unlike other hydrogel materials, have the added advantage of having high oxygen permeability, which is believed to be healthier for the eye compared to silicone-free hydrogel contact lenses. While silicone hydrogel contact lenses are typically highly lubricious and comfortable when initially worn, they often become less comfortable as wearing time progresses. This decreased discomfort may be due in part to changes in the physical properties of the surface of the contact lens. For example, many commercial contact lenses are packaged in packaging solutions that contain comfort polymers or wetting agents. However, during wear these comfort agents become washed away by the wearer's tears. Additionally, upon removal from its packaging solution and exposure to air, hydrophobic groups within the silicone hydrogel matrix may migrate toward the surface of the lens, resulting in non-wettable regions of the lens. There is a need for silicone hydrogel contact lenses that remain comfortable during the entire length of lens wearing time.

Background publications include U.S. Pat. Appl. Publ. No. 2008/0110770, U.S. Pat. Appl. Publ. No. 2014/0102917, U.S. Pat. No. 6,440,366, U.S. Pat. No. 6,867,172, and U.S. Pat. No. 8,647,658.

### SUMMARY

In one aspect, the invention provides an unworn sterile silicone hydrogel contact lens immersed in a packaging solution and sealed in a package, wherein the packaging solution comprises an ophthalmically-acceptable concentration of from about 0.01 wt.% up to about 0.5 wt.% of an alkyl aryl-containing surfactant (based on the total weight of the packaging solution), wherein the silicone hydrogel contact lens has a lower coefficient of friction after an overnight wash compared to a control lens. In one example, the alkyl aryl-containing surfactant is tyloxapol. In a specific example, the contact lens has a lower surface concentration of silicon compared to a control lens, as determined by cold stage X-ray photoelectron spectroscopy (XPS).

In another aspect, the invention provides an unworn sterile silicone hydrogel contact lens immersed in a packaging solution and sealed in a package, wherein the packaging solution comprises an ophthalmically-acceptable concentration of from about 0.01 wt.% up to about 0.5 wt.% of a poly(ethylene oxide) (PEO)-containing surfactant having a molecular weight of up to about 6,000 daltons, wherein the silicone hydrogel contact lens advantageously has a lower coefficient of friction after an overnight wash compared to a control lens.

Another aspect of the invention is a method of manufacturing a silicone hydrogel contact lens comprising curing a polymerizable composition comprising at least one silicone monomer and at least one hydrophilic monomer to form a polymeric lens body; packaging the polymeric lens body with a packaging solution comprising a surfactant, wherein the surfactant is a poly(ethylene oxide) (PEO)-containing surfactant having a molecular weight of up to about 6,000 daltons, or is an alkyl aryl-containing surfactant, or is a surfactant that is both alkyl aryl-containing and PEO-containing, or is a combination thereof; and heating the packaged polymeric lens body to provide an unworn, sterilely packaged silicone hydrogel contact lens. In a specific example, the concentration of the surfactant in the packaging solution is from about 0.01 wt.% up to about 0.5 wt.% prior to contact with the polymeric lens body.

Another aspect of the invention is a contact lens package comprising a base member having a cavity for accommodating a packaging solution and a contact lens; a silicone hydrogel contact lens in the cavity of the base member; and a packaging solution in the cavity of the base member wherein the contact lens is immersed in the packaging solution and the contact lens immersed in the packaging solution is as defined above. The silicone hydrogel contact lens can have a coefficient of friction less than 0.09 at a constant sliding speed of 0.5 mm/sec at a constant load of 0.5 g for 12 seconds at a temperature of about 20 to 25 degrees C.

### DETAILED DESCRIPTION

Highly lubricious silicone hydrogel contact lenses and their method of manufacture are described herein. The silicone hydrogel contact lenses remain lubricious after prolonged wear, for example up to 8 or 10 hours of continuous wear or longer, providing a more comfortable contact lens wearing experience. The contact lens is provided in an aqueous packaging solution comprising an ophthalmically-acceptable concentration of from about 0.01 wt.% up to about 0.5 wt.% of a surfactant, wherein the surfactant is a poly(ethylene oxide) (PEO)-containing surfactant having a molecular weight of up to about 6,000 daltons, or is an alkyl aryl-containing surfactant, or is a surfactant that is both alkyl aryl-containing and PEO-containing, or is a combination thereof. As used herein, the term "weight percent" (wt.%) is intended to refer to the percentage of weight a particular ingredient contributes to a formulation. Thus, for example, when a wt.% is given for a surfactant, it is to be understood that this is based upon the total weight of the packaging solution that contains the surfactant. Further, references to a wt.% of a particular class of ingredient (e.g. surfactant) refers to the sum of the wt.% of all ingredients of the same type.
Thus for example, a packaging solution that comprises 0.1 wt.% of a first type of alkyl aryl-containing surfactant, and 0.2 wt.% of a second type of an alkyl aryl-containing surfactant is said to comprise 0.3 wt.% of an alkyl aryl-containing surfactant. The contact lens advantageously has a lower coefficient of friction after an overnight wash compared to a control lens. The term "coefficient of friction" refers to the kinetic (dynamic) coefficient of friction (CoF) of a contact lens as measured using a CETR Universal Micro-Tribometer (UMT) or equivalent using the method described in Example 1 or equivalent. As used herein, an "overnight wash" is one in which a lens is removed from its packaging solution and soaked in 4 mL PBS for approximately 15 hours at 20° C to 25° C (i.e. room temperature). In one example, the contact lens has a coefficient of friction that is at least 50% lower than that of the control lens. In various examples, the contact lens has a coefficient of friction that is at least 10%, 25%, or 50% and up to 99% lower than that of the control lens. In a further example, the control lens has a coefficient of friction (after overnight wash) of at least 0.5. In various examples, the control lens has a coefficient of friction of at least 0.25, 0.5 and up to about 1.0, 1.5, or 1.8. As used herein, a "control lens" refers to a contact lens that has not been contacted with the surfactant but is otherwise identical to the contact lens (i.e. test lens) to which it is being compared in that it was manufactured using the same contact lens formulation (referred to herein as a "polymerizable composition"), subjected to the same manufacturing processes, and subjected to the same overnight wash procedure prior to CoF measurement.

As used herein, an alkyl aryl-containing surfactant refers to a surfactant comprising a hydrophobic portion containing at least one aryl group and at least one hydrocarbon chain of at least 2 carbon atoms. Each of the alkyl and aryl groups may be substituted or unsubstituted. In specific examples, the hydrocarbon chain comprises a chain of at least 2 or 3 carbon atoms and up to about 5, 10, 15 or 20 carbon atoms. In some examples, the alkyl aryl-containing surfactant is an oligomer of another alkyl aryl-containing surfactant. For example, tyloxapol (CAS No. 25301-02-04) is an oligomer of the alkyl aryl-containing surfactant Triton X-100 (CAS No. 9002-93-1). As used herein, a PEO-containing surfactant refers to a surfactant comprising at least one poly(ethylene oxide) segment having the formula 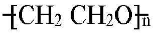 where n is an integer of at least 3. In various examples, n of the PEO segment is an integer of at least 3, 5 or 10, and up to about 25, 75 or 100. The surfactant may be nonionic or ionic (i.e. anionic, cationic, or zwitterionic). In a specific example, the surfactant is nonionic. In one example, the surfactant is both an alkyl aryl-containing and a polyethylene oxide-containing surfactant. In a specific example, the alkyl aryl-containing and polyethylene oxide-containing surfactant is tyloxapol, or a tocopheryl polyethylene glycol succinate (TPGS) (e.g. D-α-Tocopheryl polyethylene glycol 1000 succinate (CAS 9002-96-4), referred to herein as "TPGS-1000"), or a combination thereof. An additional advantage of tyloxapol and TPGS is that they exhibit antioxidant activity, which may be beneficial for ocular health.

Without being limited by any particular theory, it is believed that the surfactants described herein may exert chaotropic activity, which changes the molecular structure of the silicone hydrogel matrix resulting in fewer hydrophobic silicone groups on the lens surface compared to a control lens. In some examples, the silicone hydrogel contact lens of the present invention has a surface concentration of elemental silicon that is at least 10% lower compared to a control lens. As used herein, surface concentration of silicon is determined by cold stage X-ray photoelectron spectroscopy (XPS) using the method described in Example 2 below, or equivalent method. In one example, the contact lens has a surface concentration of silicon that is at least 20% lower, at least 30% lower, or at least 40% lower than the surface concentration of silicone of a control lens.

As used herein, an "ophthalmically-acceptable concentration" means a concentration of the surfactant in the packaging solution (prior to its contact with the contact lens) that does not exhibit cytotoxicity as determined by an ocular irritation study conducted in accordance with ISO 10993-10. An advantage of the surfactants used herein is that they are non-cytotoxic at concentrations effective at reducing surface concentration of silicon, and thus may be left in the final packaged contact lens. In contrast, other methods for reducing surface concentration of silicone of silicone hydrogel contact lenses employ toxic compounds that must be extracted from the contact lens prior to final packaging (see e.g. US Pat. Appln. Publ. No. 2014/0275434). Thus, the prior art method requires additional processing steps in order to provide an ophthalmically-acceptable contact lens, which add to manufacturing costs. Further, there is a desire to minimize the use of hazardous chemicals in manufacturing sites, which is another advantage of the present invention compared to the prior art. In specific examples, the concentration of the surfactant in the packaging solution prior to contact with the contact lens is from about 0.01 wt.% or 0.02 wt.%, up to about 0.05 wt.%, 0.075 wt.%, or 0.1 wt.%. Throughout this disclosure, when a series of lower limit ranges and a series of upper limit ranges are provided, all combinations of the provided ranges are contemplated as if each combination were specifically listed. For example, in the above listing of surfactant concentrations, all six possible concentration ranges are contemplated (i.e. 0.01 wt.% to 0.05 wt.%, 0.01 wt.%. to 0.1 wt.%, etc. and 0.02 wt.% to 0.1 wt.%). Also, throughout this disclosure, when a series of values is presented with a qualifier preceding the first value, the qualifier is intended to implicitly precede each value in the series unless context dictates otherwise. For example, for the values listed above, it is intended that the qualifier "from about" implicitly precedes the values 0.02, and that the qualifier "up to about" implicitly precedes each of the values 0.075 and 0.1. In a specific example, the packaging solution comprises from about 0.01 wt.% to about 0.1 wt.% tyloxapol.

In addition to the surfactant, the packaging solution typically comprises a buffered saline solution such as phosphate- or borate-buffered saline. The packaging solution may optionally contain additional ingredients such as a comfort agent, a hydrophilic polymer, an additive that prevents the lens from sticking to the container, and/or a chelating agent, etc. In some examples, the packaging solution is substantially free of polysaccharides (e.g. hyaluronic acid, hydroxypropyl methylcellulose, hydroxypropyl cellulose, hydroxyethyl cellulose, etc.) or other high molecular weight polymers, such as polyvinyl pyrrolidone, which are commonly used as comfort polymers or thickening agents in ophthalmic solutions and contact lens packaging solutions. As used herein, a high molecular weight polymer refers to a polymer having an average molecular weight of at least 50,000 daltons.

The PEO- or alkyl aryl-containing surfactant will typically have a molecular weight of less than about 6,000. In one example, the PEO- or alkyl aryl-containing surfactant has a molecular weight of less than about 5,000. The term "molecular weight" as used herein, refers to the absolute number average molecular weight (in units of daltons) of the surfactant as determined by ¹H NMR end-group analysis. In a specific example, the surfactant has a molecular weight of about 1,000 to about 5,000. In other examples, the surfactant has a molecular weight of at least 500, 750, or 1,000, up to about 4,000, 5,000, or 6,000. In a specific example, every ingredient of the packaging solution has a molecular weight less than 6,000 daltons.

In various examples, the PEO- or alkyl aryl-containing surfactant has a hydrophilic-lipophilic balance (HLB) value that is at least 10, 11, or 12, up to about 14, 15, or 16, where the HLB value is calculated as twenty times the molecular weight of the hydrophilic portion of the polysiloxane divided by the total molecular weight of the polysiloxane. Both tyloxapol and TPGS-1000 have HLB values of about 13.

The contact lens is provided unworn (i.e. it is a new contact lens, not having been previously used by a patient), immersed in the packaging solution and sealed in a package. The package may be a blister package, glass vial, or other appropriate container. The package comprises a base member having a cavity for accommodating a packaging solution and an unworn silicone hydrogel contact lens. The sealed package may be sterilized by sterilizing amounts of radiation, including heat or steam, such as by autoclaving, or by gamma radiation, e-beam radiation, ultraviolet radiation, etc. In a specific example, the packaged contact lens is sterilized by autoclaving. The final product is a sterile, packaged ophthalmically-acceptable contact lens. In a specific example, the invention provides a contact lens package, comprising a base member having a cavity for accommodating a packaging solution and a contact lens; an unworn silicone hydrogel contact lens in the cavity of the base member; and a packaging solution in the cavity of the base member, wherein the packaging solution comprises from about 0.01 wt.% to about 0.1 wt.% tyloxapol.

Another aspect of the invention is a method of manufacturing an ophthalmically-acceptable silicone hydrogel contact lens. The method comprises curing a polymerizable composition comprising at least one silicone monomer and at least one hydrophilic monomer or hydrophilic polymer to form a polymeric lens body, and packaging the polymeric lens body with a packaging solution comprising a surfactant, wherein the surfactant is a poly(ethylene oxide) (PEO)-containing surfactant having a molecular weight of up to about 6,000 daltons, or is an alkyl aryl-containing surfactant, or is a surfactant that is both alkyl aryl-containing and PEO-containing, or is a combination thereof. The packaging solution has a concentration of the surfactant of from about 0.01% to about 0.1% prior to contact with polymeric lens body. The method further comprises heating (e.g.autoclaving) the packaged polymeric lens body to provide an unworn, sterilely packaged contact lens which advantageously has a lower coefficient of friction after an overnight wash compared to a control lens that has not been contacted with the surfactant but is otherwise identical.

The polymerizable composition comprises at least one siloxane monomer and at least one hydrophilic monomer or at least one hydrophilic polymer, or a combination thereof. As used herein, the term "siloxane monomer" is a molecule that contains at least one Si-O group and at least one polymerizable group. Siloxane monomers useful in contact lens compositions are well-known in the art (see, e.g., US Pat No. 8,658,747 and US Pat No. 6,867,245). In some examples, the polymerizable composition comprises a total amount of siloxane monomer of at least 10 wt.%, 20 wt.%, or 30 wt.% up to about 40 wt.%, 50 wt.%, 60 wt.%, or 70 wt.%. Unless specified otherwise, as used herein, a given weight percentage (wt. %) of a component of the polymerizable composition is relative to the total weight of all polymerizable ingredients and IPN polymers (as described further below) in the polymerizable composition. The weight of the polymerizable composition contributed by components, such as diluents, that do not incorporate into the final contact lens product are not included in the wt.% calculation.

In a specific example, the polymerizable composition comprises a hydrophilic vinyl monomer. As used-herein, a "hydrophilic vinyl monomer" is any siloxane-free (i.e. contains no Si-O groups) hydrophilic monomer having a polymerizable carbon-carbon double bond (i.e., a vinyl group) present in its molecular structure that is not part of an acryl group, where the carbon-carbon double bond of the vinyl group is less reactive than the carbon-carbon double bond present in a polymerizable methacrylate group under free radical polymerization. As used herein, the term "acryl group" refers to the polymerizable group present in acrylate, methacrylates, acrylamides, etc. Thus, while carbon-carbon double bonds are present in acrylate and methacrylate groups, as used herein, such polymerizable groups are not considered to be vinyl groups. Further, as used herein, a monomer is "hydrophilic" if at least 50 grams of the monomer are fully soluble in 1 liter of water at 20°C (i.e., ∼ 5% soluble in water) as determined visibly using a standard shake flask method. In various examples, the hydrophilic vinyl monomer is N-vinyl-N-methylacetamide (VMA), or N-vinyl pyrrolidone (NVP), or 1,4-butanediol vinyl ether (BVE), or ethylene glycol vinyl ether (EGVE), or diethylene glycol vinyl ether (DEGVE), or any combination thereof. In one example, the polymerizable composition comprises at least 10 wt.%, 15 wt.%, 20 wt.%, or 25 wt.% up to about 45 wt.%, 60 wt.%, or 75 wt.% of a hydrophilic vinyl monomer. As used herein, a given weight percentage of a particular class of component (e.g., hydrophilic vinyl monomer, siloxane monomer, or the like) in the polymerizable composition equals the sum of the wt.% of each ingredient in the composition that falls within the class. Thus, for example, a polymerizable composition that comprises 5 wt.% BVE and 25 wt.% NVP and no other hydrophilic vinyl monomer, is said to comprise 30 wt.% hydrophilic vinyl monomer. In one example, the hydrophilic vinyl monomer is a vinyl amide monomer. Exemplary hydrophilic vinyl amide monomers are VMA and NVP. In a specific example, the polymerizable composition comprises at least 25 wt.% of a vinyl amide monomer. In a further specific example, the polymerizable composition comprises from about 25 wt.% up to about 75 wt.% of VMA or NVP, or a combination thereof. Additional hydrophilic monomers that may be included in the polymerizable composition are N,N-dimethylacrylamide (DMA), 2-hydroxyethyl methacrylate (HEMA), ethoxyethyl methacrylamide (EOEMA), ethylene glycol methyl ether methacrylate (EGMA), and combinations thereof.

In addition or as an alternative to a hydrophilic monomer, the polymerizable composition may comprise a non-polymerizable hydrophilic polymer, which results in a polymeric lens body comprising an interpenetrating polymer network (IPN) with the non-polymerizable hydrophilic polymer interpenetrating the silicone hydrogel polymer matrix. In this example, the non-polymerizable hydrophilic polymer is referred to as an IPN polymer, which acts as an internal wetting agent in the contact lens. In contrast, polymer chains within the silicone hydrogel network that form by polymerization of monomers present in the polymerizable composition are not considered to be IPN polymers. The IPN polymer may be a high molecular weight hydrophilic polymer, for example from about 50,000 to about 500,000 daltons. In a specific example, the IPN polymer is polyvinylpyrrolidone (PVP). In other examples, the polymerizable composition is substantially free of polyvinyl pyrrolidone or other IPN polymer.

The polymerizable composition may additionally comprise at least one cross-linking agent. As used herein, a "cross-linking agent" is a molecule having at least two polymerizable groups. Thus, a cross-linking agent can react with functional groups on two or more polymer chains so as to bridge one polymer to another. The cross-linking agent may comprise an acryl group or a vinyl group, or both an acryl group and a vinyl group. In certain examples, the cross-linking agent is free of siloxane moieties, i.e., it is a non-siloxane cross-linking agent. A variety of cross-linking agents suitable for use in silicone hydrogel polymerizable compositions are known in the field (see, e.g., U.S. Pat. No. 8,231,218. Examples of suitable cross-linking agents include, without limitation, lower alkylene glycol di(meth)acrylates such as triethylene glycol dimethacrylate and diethylene glycol dimethacrylate; poly(lower alkylene) glycol di(meth)acrylates; lower alkylene di(meth)acrylates; divinyl ethers such as triethyleneglycol divinyl ether, diethyleneglycol divinyl ether, 1,4-butanediol divinyl ether and 1,4-cyclohexanedimethanol divinyl ether; divinyl sulfone; di- and trivinylbenzene; trimethylolpropane tri(meth)acrylate; pentaerythritol tetra(meth)acrylate; bisphenol A di(meth)acrylate; methylenebis(meth)acrylamide; triallyl phthalate; 1,3-Bis(3-methacryloxypropyl)tetramethyldisiloxane; diallyl phthalate; and combinations thereof.

As will be appreciated by those skilled in the art, the polymerizable composition may comprise additional polymerizable or non-polymerizable ingredients conventionally used in contact lens formulations such as one or more of a polymerization initiator, a UV absorbing agent, a tinting agent, an oxygen scavenger, a chain transfer agent, or the like. In some examples, the polymerizable composition may include an organic diluent in an amount to prevent or minimize phase separation between the hydrophilic and hydrophobic components of the polymerizable composition, so that an optically clear lens is obtained. Diluents commonly used in contact lens formulations include hexanol, ethanol, and/or other alcohols. In other examples, the polymerizable composition is free or substantially free (e.g., less than 500 ppm) of an organic diluent. In such examples, the use of siloxane monomers containing hydrophilic moieties such as polyethylene oxide groups, pendant hydroxyl groups, or other hydrophilic groups, may make it unnecessary to include a diluent in the polymerizable composition. Non-limiting examples of these and additional ingredients that may be included in the polymerizable composition are provided in U.S. Pat. No. 8,231,218.

The polymerizable composition is dispensed into a contact lens mold and cured using conventional methods (e.g. thermal curing or UV-curing) to provide a polymeric lens body. The polymeric lens body is typically removed from the mold and washed, typically with alcohol and/or water, to remove unreacted components and hydrate the lens. The lens is then placed in a package with a volume of the packaging solution comprising the PEO- or alkyl aryl-containing surfactant, sealed, and heated (e.g. autoclaved). The packaging solution of the final product will have a concentration of the surfactant that is less than the concentration of the surfactant prior to the heating step. In a specific example, the packaging solution has a concentration of the surfactant that is at least 20% less than the concentration of the surfactant prior to the heating step.

The following Examples illustrate certain aspects and advantages of the present invention, which should be understood not to be limited thereby.

### Example 1: Surfactant Screening

Silicone hydrogel contact lenses designated "Lens-1" in Table 1 below, were prepared from a polymerizable composition similar to composition number 9 described in Table I of U.S. Pat. No. 8,614,261. The polymerizable composition comprised about 40 wt.% of a combination of NVP and VMA, about 55 wt.% of a combination of silicone monomers, as well as methacrylate-containing monomers, a cross-linking agent, and a polymerization initiator. The polymerizable composition was UV-cured in polar molds, and the resulting lens bodies were extracted in ethanol, and rinsed in water.

Silicone hydrogel contact lenses designated "Lens-2" in Table 1 below, were prepared from a polymerizable composition similar to the compositions described in Example 2 of U.S. Pat. No. 9,164,298. The polymerizable composition comprised about 40 wt.% VMA, about 35 wt.% of a combination of silicone monomers, as well methacrylate-containing monomers, a cross-linking agent, and a polymerization initiator. The polymerizable composition was thermally-cured in non-polar molds, and the resulting lens bodies were extracted in ethanol, and rinsed in water.

The contact lenses were soaked overnight in 3 mL of PBS with or without the commercially-available surfactant designated in Table 1 below. All surfactants were used at a concentration of 0.1 wt.%, except for coco betaine and PEG-7 glyceryl cocoate, which were used at a concentration of 0.5 wt.%. The lenses were removed from the surfactants and soaked in 4 mL PBS overnight at room temperature.

The kinetic (dynamic) coefficient of friction (CoF) of the contact lenses were measured using a CETR Universal Micro-Tribometer (UMT) and CETR UMT Multi-Specimen Testing System software, with a pin-on-disk sample mount at ambient temperature. An adhesive-backed, 2.5" round polyethylene terephthalate film was adhered to the rotational disk, which is mounted on the mounting ring of the UMT. Each contact lens was picked up with tweezers and mounted onto the sample holder. 100 µL PBS was dispensed onto the PET substrate under the lens holder. The center of the lens on the pin tip was pressed against the PBS-wetted PET film moving at a constant sliding speed of 0.5 mm/sec at a constant load of 0.5 g for 12 seconds at a temperature between about 20 degrees C and 25 degrees C. CoF values were computed by the software, and the average values (n=3) for each lens is shown in Table 1.

**Table 1: Surfactant Screening**

| **Surfactant** | **Lens-1 CoF** | **Lens-2 CoF** |
|---|---|---|
| Control | 0.52 | 1.30 |
| Tyloxapol | 0.09 | 0.23 |
| Tetronic 1107 | 0.17 | 1.39 |
| Coco betaine | 0.29 | 0.78 |
| PEG-7 glyceryl cocoate | 0.09 | 0.75 |
| Zwittergent 3-10 | 0.98 | 1.08 |
| Poloxamer 407 | 0.18 | 0.33 |
| Tetronic 904 | 0.07 | 0.91 |
| Kolliphor P188 | 0.44 | 0.39 |
| Kolliphor RH 40 | 0.04 | 0.53 |
| Plantapon LGC Sorb | 0.05 | 0.56 |
| Plantapon LC 7 | 0.05 | 0.37 |
| RLM-100 | 0.03 | 0.08 |
| D-α-Tocopheryl polyethlene glycol 1000 succinate | 0.05 | 0.37 |

Because of the low CoF values obtained for both lenses, tyloxapol and RLM-100 were evaluated further for use as a possible packaging solution additive. Titration studies showed that tyloxapol at a concentration of 0.02 wt.% remained effective in reducing CoF of silicone hydrogel contact lenses after an overnight wash. A concentration of 0.035% tyloxapol in PBS was tested for cytotoxicity by an ocular irritation study in rabbits (ISO 10993-10), and was shown to be non-cytotoxic. In contrast, RLM-100 was cytotoxic at a concentration of 0.01 wt.%, and at this concentration was no longer effective in reducing CoF of the silicone hydrogel contact lenses.

### Example 2: XPS Surface Analysis of Contact Lenses Packaged in 0.02 wt.% Tyloxapol.

Silicone hydrogel contact lenses were prepared from a polymerizable composition similar to the compositions described in Table I of U.S. Pat. No. 8,614,261. The polymerizable composition comprised about 40 wt.% of a combination of NVP and VMA, about 55 wt.% of a combination of silicone macromers, as well as methacrylate-containing monomers, a cross-linking agent, and a polymerization initiator. The polymerizable composition was UV-cured in polar molds, and the resulting lens bodies were extracted in ethanol, rinsed in water, sealed in blister packages containing about 1.8 ml PBS with or without 0.02wt% tyloxapol, and autoclaved.

Two contact lenses (test-1, and test-2) that had been autoclaved in the tyloxapol-containing packaging solution and a control lens (packaged in PBS without tyloxapol) were tested by XPS. Each contact lens sample was rinsed in ultra-pure water three times for 2 minutes, 10 minutes, and then 2 minutes, with the volume of water replaced after each rinsing cycle. Each sample was mounted whole on a dome-shaped mount and placed in the intro chamber of a PHI 5802 Multitechnique XPS system. A droplet of ultra-pure water was placed on the center of the lens prior to freezing. Then the samples were frozen (using liquid nitrogen) in the intro chamber before the initial pumpdown (with nitrogen gas flow). The ice was sublimated while pumping down the intro chamber to preserve the hydrated state of the surface. Once the ice was sublimated, the samples were introduced into the analytical chamber of the instrument. The spectral acquisitions were performed while the sample stage was constantly cooled by liquid nitrogen. The analytical parameters for the XPS analysis are shown in Table 2.

**Table 2: Analytical Parameters**

| | |
|---|---|
| **X-ray source** | Monochromatic AI K_{α} s1486.6eV |
| **Acceptance Angle** | ±23 ° |
| **Take-off Angle** | 45° |
| **Analysis Area** | 800µm |
| **Charge Correction** | C-C, H, Si in C1s set to 284.8eV |
| **Charge Compensation** | Electron and Ion floods |
| **Cold Stage Sample Temperature** | -50°C... -100°C |

The analysis depth ranged from about 50 Ǻ to about 100 Ǻ. The atomic concentrations of elements detected are shown in Table 3. The concentration values are normalized to 100% of the elements detected (XPS does not detect H).

**Table 3: Atomic Concentrations (in %)**

| **Sample** | **C1s** | **N1s** | **O1s** | **F1s** | **Si2p** |
|---|---|---|---|---|---|
| **Control** | 61.1 | 4.8 | 22.9 | 1.0 | 10.1 |
| **Test-1** | 67.1 | 7.0 | 20.0 | 0.5 | 5.4 |
| **Test-2** | 67.0 | 7.6 | 20.1 | 0.4 | 4.9 |

### Example 3: Reduced Coefficient of Friction of Commercial Lenses Autoclaved in 0.02% Tyloxapol

Commercial contact lenses were removed from their original blisters and CoF was measured using the method described in Example 1. The lenses were then vortexed in 20 mL PBS three times, soaked overnight in 20 mL PBS, repackaged in 1.8 ml of either PBS (control) or PBS with 0.02wt% tyloxapol, and autoclaved. After autoclave, the Cof values of the repackaged contact lenses were measured. The results are shown in Table 4.

**Table 4**

| **Contact Lens** | | **CoF** | | |
|---|---|---|---|---|
| **Brand** | **Material** | **Blister** | **Control** | **Tyloxapol** |
| Oasys | senofilcon A | 0.05 | 1.31 | 0.09 |
| TruEye | narafilcon A | 0.07 | 0.38 | 0.09 |
| Clariti | somofilcon A | 0.14 | 2.45 | 0.15 |
| Biofinity | comfilcon A | 0.07 | 1.26 | 0.03 |
| MyDay | stenfilcon A | 0.21 | 0.66 | 0.06 |

The disclosure herein refers to certain illustrated examples, it is to be understood that these examples are presented by way of example and not by way of limitation.

## Claims

1. An unworn sterile silicone hydrogel contact lens immersed in a packaging solution and sealed in a package, wherein the packaging solution comprises:
(a) an ophthalmically-acceptable concentration of from 0.01 wt.% up to 0.5 wt.% of an alkyl aryl-containing surfactant; or
(b) an ophthalmically-acceptable concentration of from 0.01 wt.% up to 0.5 wt.% of a poly(ethylene oxide) (PEO)-containing surfactant having an absolute number average molecular weight of up to 6,000 daltons,
wherein the contact lens has a lower coefficient of friction after an overnight wash compared to a control lens that has not been contacted with the alkyl aryl-containing or PEO-containing surfactant but is otherwise identical.

2. The contact lens of claim 1, wherein the alkyl aryl-containing surfactant comprises polyethylene oxide, or wherein the PEO-containing surfactant is an alkyl aryl polyethylene oxide.

3. The contact lens of claim 1 or claim 2, wherein the alkyl aryl-containing surfactant has an absolute number average molecular weight of up to 6,000 daltons.

4. The contact lens of any preceding claim, wherein the alkyl aryl-containing surfactant and/or the PEO-containing surfactant has an HLB value of less than 16.

5. The contact lens of any preceding claim, wherein the alkyl aryl-containing surfactant and/or the PEO-containing surfactant is tyloxapol, or a tocopheryl polyethylene glycol succinate, or a combination thereof.

6. The contact lens of claim 1 or claim 2, wherein the concentration of the alkyl aryl-containing surfactant and/or the PEO-containing surfactant is from 0.01 wt.% to 0.1 wt.%.

7. The contact lens of any preceding claim having a lower surface concentration of silicon compared to the control lens, wherein the surface concentration of silicon is determined by cold stage X-ray photoelectron spectroscopy (XPS), and wherein the surface concentration of silicon is optionally at least 20% lower than the control lens.

8. The contact lens of any preceding claim, wherein the packaging solution comprises from about 0.01 wt.% to about 0.1 wt.% tyloxapol.

9. The contact lens of any preceding claim, wherein the packaging solution is free of a polymer having an absolute number average molecular weight of at least 50,000 daltons.

10. The contact lens of any one of claims 1 to 9, wherein every ingredient in the packaging solution has an absolute number average molecular weight of less than 6,000 daltons.

11. A contact lens package comprising:
a base member having a cavity for accommodating a packaging solution and a contact lens;
an unworn silicone hydrogel contact lens in the cavity of the base member; and
a packaging solution in the cavity of the base member,
wherein the contact lens is immersed in the packaging solution and the contact lens immersed in the packaging solution is as defined in any preceding claim.

12. The contact lens package of claim 11, wherein the silicone hydrogel contact lens comprises a reaction product of a polymerizable composition comprising a hydrophilic vinyl monomer or a polymerizable composition comprising at least 25 wt.% of a vinyl amide monomer, and/or wherein the silicone hydrogel contact lens comprises a reaction product of a polymerizable composition that is free of a polyvinyl pyrrolidone.

13. The contact lens package of claim 11 or claim 12, wherein the silicone hydrogel contact lens has a coefficient of friction less than 0.09 at a constant sliding speed of 0.5 mm/sec at a constant load of 0.5 g for 12 seconds at a temperature of 20 to 25 degrees C.

14. A method of manufacturing the unworn sterile silicone hydrogel contact lens immersed in the packaging solution of any one of claims 1 to 10 comprising:
a) curing a polymerizable composition comprising at least one silicone monomer and at least one hydrophilic monomer to form a polymeric lens body;
b) packaging the polymeric lens body with the packaging solution, wherein the packaging solution has a concentration of the alkyl aryl-containing surfactant or the PEO-containing surfactant of from 0.01 wt.% up to 0.5 wt.% prior to contact with polymeric lens body; and
c) heating the packaged polymeric lens body to provide the unworn, sterilely packaged contact lens,
wherein the contact lens has a lower coefficient of friction after an overnight wash compared to a control lens that has not been contacted with the alkyl aryl-containing surfactant but is otherwise identical.

15. The package of any one of claims 11 to 13 or the method of claim 14, wherein the polymerizable composition comprises N-vinyl-N-methylacetamide (VMA), or N-vinyl pyrrolidone (NVP), or 1,4-butanediol vinyl ether (BYE), or ethylene glycol vinyl ether (EGVE), or diethylene glycol vinyl ether (DEGVE), or a combination thereof

16. The method of claim 14 or claim 15, wherein the polymerizable composition comprises at least 25 wt.% of a vinyl amide monomer, or wherein the polymerizable composition is free of a polyvinyl pyrrolidone.

17. The method of any one of claims 14 to 16, wherein after the heating step, the packaging solution has a concentration of the PEO-containing surfactant that is at least 20% less than the concentration of the PEO-containing prior to contact with polymeric lens body.

## Patentansprüche

1. Ungetragene sterile Silikonhydrogel-Kontaktlinse, die in eine Verpackungslösung eingetaucht und in einer Verpackung versiegelt ist, wobei die Verpackungslösung umfasst:
(a) eine ophthalmisch akzeptable Konzentration von 0,01 Gew.-% bis zu 0,5 Gew.-% eines Alkylaryl enthaltenden Tensids; oder
(b) eine ophthalmisch akzeptable Konzentration von 0,01 Gew.-% bis zu 0,5 Gew.-% eines Poly(ethylenoxid) (PEO) enthaltenden Tensids mit einem absoluten Zahlenmittel des Molekulargewichts von bis zu 6.000 Dalton,
wobei die Kontaktlinse nach einer Wäsche über Nacht einen niedrigeren Reibungskoeffizienten aufweist, verglichen mit einer Kontrolllinse, die nicht mit dem Alkylaryl enthaltenden oder PEO enthaltenden Tensid in Kontakt gebracht wurde, ansonsten jedoch identisch ist.

2. Kontaktlinse nach Anspruch 1, wobei das Alkylaryl enthaltende Tensid Polyethylenoxid umfasst oder wobei das PEO enthaltende Tensid ein Alkylarylpolyethylenoxid ist.

3. Kontaktlinse nach Anspruch 1 oder Anspruch 2, wobei das Alkylaryl enthaltende Tensid ein absolutes Zahlenmittel des Molekulargewichts von bis zu 6.000 Dalton aufweist.

4. Kontaktlinse nach einem der vorhergehenden Ansprüche, wobei das Alkylaryl enthaltende Tensid und/oder das PEO enthaltende Tensid einen HLB-Wert von weniger als 16 aufweist.

5. Kontaktlinse nach einem der vorhergehenden Ansprüche, wobei das Alkylaryl enthaltende Tensid und/oder das PEO enthaltende Tensid Tyloxapol oder ein Tocopheryl-Polyethylenglykolsuccinat oder eine Kombination davon ist.

6. Kontaktlinse nach Anspruch 1 oder Anspruch 2, wobei die Konzentration des Alkylaryl enthaltenden Tensids und/oder des PEO enthaltenden Tensids 0,01 Gew.-% bis 0,1 Gew.-% beträgt.

7. Kontaktlinse nach einem der vorhergehenden Ansprüche, die im Vergleich zur Kontrolllinse eine niedrigere Oberflächenkonzentration von Silizium aufweist, wobei die Oberflächenkonzentration von Silizium durch Kaltstufen-Röntgen-Photoelektronen-Spektroskopie (XPS) bestimmt wird und wobei die Oberflächenkonzentration von Silizium optional mindestens 20% niedriger ist als bei der Kontrolllinse.

8. Kontaktlinse nach einem der vorhergehenden Ansprüche, wobei die Verpackungslösung etwa 0,01 Gew.-% bis etwa 0,1 Gew.-% Tyloxapol umfasst.

9. Kontaktlinse nach einem der vorhergehenden Ansprüche, wobei die Verpackungslösung kein Polymer mit einem absoluten Zahlenmittel des Molekulargewichts von mindestens 50.000 Dalton aufweist.

10. Kontaktlinse nach einem der Ansprüche 1 bis 9, wobei jeder Inhaltsstoff in der Verpackungslösung ein absolutes Zahlenmittel des Molekulargewichts von weniger als 6.000 Dalton aufweist.

11. Kontaktlinsenpackung, umfassend:
ein Basiselement mit einem Hohlraum zur Aufnahme einer Verpackungslösung und einer Kontaktlinse;
eine ungetragene Silikon-Hydrogel-Kontaktlinse im Hohlraum des Basiselements; und
eine Verpackungslösung im Hohlraum des Basiselements,
wobei die Kontaktlinse in die Verpackungslösung eingetaucht ist und die in die Verpackungslösung eingetauchte Kontaktlinse wie in einem der vorhergehenden Ansprüche definiert ist.

12. Kontaktlinsenpackung nach Anspruch 11, wobei die Silikonhydrogelkontaktlinse ein Reaktionsprodukt einer polymerisierbaren Zusammensetzung, die ein hydrophiles Vinylmonomer umfasst, oder einer polymerisierbaren Zusammensetzung umfasst, die mindestens 25 Gew.-% eines Vinylamidmonomers umfasst, und/oder wobei die Silikon-Hydrogel-Kontaktlinse ein Reaktionsprodukt einer polymerisierbaren Zusammensetzung umfasst, die kein Polyvinylpyrrolidon enthält.

13. Kontaktlinsenpackung nach Anspruch 11 oder 12, wobei die Silikonhydrogel-Kontaktlinse bei einer konstanten Gleitgeschwindigkeit von 0,5 mm/s bei einer konstanten Last von 0,5 g für 12 Sekunden bei einer Temperatur von 20 bis 25 ° C einen Reibungskoeffizienten von weniger als 0,09 aufweist.

14. Verfahren zur Herstellung der ungetragenen, sterilen Silikonhydrogelkontaktlinse, die in die Verpackungslösung nach einem der Ansprüche 1 bis 10 eingetaucht ist, umfassend:
a) Härten einer polymerisierbaren Zusammensetzung, die mindestens ein Silikonmonomer und mindestens ein hydrophiles Monomer umfasst, um einen polymeren Linsenkörper zu bilden;
b) Verpacken des polymeren Linsenkörpers mit der Verpackungslösung, wobei die Verpackungslösung vor dem Kontakt mit dem polymeren Linsenkörper eine Konzentration des Alkylaryl enthaltenden Tensids oder des PEO enthaltenden Tensids von 0,01 Gew.-% bis 0,5 Gew.-% aufweist; und
c) Erwärmen des verpackten polymeren Linsenkörpers, um die ungetragene, steril verpackte Kontaktlinse bereitzustellen,
wobei die Kontaktlinse nach einem Waschvorgang über Nacht im Vergleich zu einer Kontrolllinse, die nicht mit dem Alkylaryl enthaltenden Tensid in Kontakt gebracht wurde, ansonsten jedoch identisch ist, einen niedrigeren Reibungskoeffizienten aufweist.

15. Packung nach einem der Ansprüche 11 bis 13 oder Verfahren nach Anspruch 14, wobei die polymerisierbare Zusammensetzung N-Vinyl-N-methylacetamid (VMA) oder N-Vinylpyrrolidon (NVP) oder 1,4-Butandiolvinylether (BVE) oder Ethylenglycolvinylether (EGVE) oder Diethylenglycolvinylether (DEGVE) oder eine Kombination davon umfasst.

16. Verfahren nach Anspruch 14 oder 15, wobei die polymerisierbare Zusammensetzung mindestens 25 Gew.-% eines Vinylamidmonomers umfasst oder wobei die polymerisierbare Zusammensetzung kein Polyvinylpyrrolidon enthält.

17. Verfahren nach einem der Ansprüche 14 bis 16, wobei die Verpackungslösung nach dem Erwärmungsschritt eine Konzentration des PEO enthaltenden Tensids aufweist, die mindestens 20% niedriger ist als die Konzentration des PEOenthaltenden Tensids vor dem Kontakt mit dem polymeren Linsenkörper.

## Revendications

1. Lentille de contact en hydrogel de silicone stérile jamais portée, immergée dans une solution de conditionnement et scellée dans un emballage, dans laquelle la solution de conditionnement comprend :
(a) une concentration acceptable sur le plan opthtalmique comprise entre 0,01 % en poids et 0,5 % en poids d'un agent tensio-actif contenant un alkyle aryle ; ou
(b) une concentration acceptable sur le plan opthtalmique comprise entre 0,01 % en poids et 0,5 % en poids d'un agent tensio-actif contenant l'oxyde de polyéthylène (PEO) ayant une masse moléculaire moyenne en nombre absolue pouvant aller jusqu'à 6 000 daltons,
dans laquelle la lentille de contact a un coefficient de friction inférieur après un lavage pendant une nuit, comparé à une lentille témoin qui n'a pas été mise en contact avec l'agent tensio-actif contenant un alkyle aryle ou l'agent tensio-actif contenant l'oxyde de polyéthylène (PEO), mais qui est par ailleurs identique.

2. Lentille de contact selon la revendication 1, dans laquelle l'agent tensio-actif contenant un alkyle aryle comprend de l'oxyde de polyéthylène, ou, dans laquelle l'agent tensio-actif contenant de l'oxyde de polyéthylène (PEO) est un oxyde de polyéthylène d'alkyle aryle.

3. Lentille de contact selon la revendication 1 ou la revendication 2, dans laquelle l'agent tensio-actif contenant un alkyle aryle a une masse moléculaire moyenne en nombre absolue pouvant aller jusqu'à 6 000 daltons.

4. Lentille de contact selon l'une quelconque des revendications précédentes, dans laquelle l'agent tensio-actif contenant un alkyle aryle et / ou l'agent tensio-actif contenant de l'oxyde de polyéthylène (PEO) a une valeur HLB inférieure à 16.

5. Lentille de contact selon l'une quelconque des revendications précédentes, dans laquelle l'agent tensio-actif contenant un alkyle aryle et / ou l'agent tensio-actif contenant de l'oxyde de polyéthylène (PEO) est du tyloxapol, ou un succinate de tocophéryl polyéthylène glycol, ou une combinaison de ceux-ci.

6. Lentille de contact selon la revendication 1 ou la revendication 2, dans laquelle la concentration de l'agent tensio-actif contenant un alkyle aryle et / ou l'agent tensio-actif contenant de l'oxyde de polyéthylène (PEO) est comprise entre 0,01 % en poids et 0,1 % en poids.

7. Lentille de contact selon l'une quelconque des revendications précédentes ayant une concentration en surface de silicone inférieure comparée à la lentille témoin, dans laquelle la concentration en surface de silicone est déterminée par spectroscopie de photoélectrons X (XPS) en phase froide, et dans laquelle la concentration en surface de silicone est facultativement au moins de 20 % inférieure à celle de la lentille témoin.

8. Lentille de contact selon l'une quelconque des revendications précédentes, dans laquelle la solution de conditionnement comprend entre environ 0,01 % en poids et environ 0,1 % en poids de tyloxapol.

9. Lentille de contact selon l'une quelconque des revendications précédentes, dans laquelle la solution de conditionnement est exempte d'un polymère ayant une masse moléculaire moyenne en nombre absolue d'au moins 50 000 daltons.

10. Lentille de contact selon l'une quelconque des revendications 1 à 9, dans laquelle tout ingrédient de la solution de conditionnement a une masse moléculaire moyenne en nombre absolue de moins de 6 000 daltons.

11. Emballage pour lentilles de contact comprenant :
un élément de base ayant une cavité pour loger une solution de conditionnement et une lentille de contact ;
une lentille de contact en hydrogel de silicone jamais portée dans la cavité de l'élément de base ; et
une solution de conditionnement dans la cavité de l'élément de base,
dans lequel la lentille de contact est immergée dans la solution de conditionnement et la lentille de contact immergée dans l solution de conditionnement est telle que définie dans l'une quelconque des revendications précédentes.

12. Emballage pour lentilles de contact selon la revendication 11, dans lequel la lentille de contact en hydrogel de silicone comprend un produit de réaction d'une composition polymérisable comprenant un vinyle monomère hydrophile ou une composition polymérisable comprenant au moins 25 % en poids d'un monomère d'amide vinylique, et / ou dans lequel la lentille de contact en hydrogel de silicone comprend un produit de réaction d'une composition polymérisable qui est exempte d'une polyvinyle pyrrolidone.

13. Emballage pour lentilles de contact selon la revendication 11 ou la revendication 12, dans lequel la lentille de contact en hydrogel de silicone a un coefficient de friction inférieure à 0,09 à une vitesse de glissement constante de 0,5 mm / s, à une charge constante de 0,5 g, pendant 12 secondes, à une température comprise entre 20 et 25 degrés C.

14. Procédé de fabrication de la lentille de contact en hydrogel de silicone stérile jamais portée, immergée dans la solution de conditionnement, selon l'une quelconque des revendications 1 à 10, comprenant :
a) le durcissement d'une composition polymérisable comprenant au moins un monomère silicone et au moins un monomère hydrophile pour former un corps de lentille en polymère ;
b) le conditionnement du corps de lentille en polymère avec la solution de conditionnement, dans lequel la solution de conditionnement a une concentration de l'agent tensio-actif contenant un alkyle aryle ou de l'agent tensio-actif contenant de l'oxyde de polyéthylène (PEO) comprise entre 0,01 % en poids et 0,5 % en poids, avant d'entrer en contact avec le corps de lentille en polymère ; et
c) le chauffage du corps de lentille en polymère emballée pour produire la lentille de contact emballée stérilement, jamais portée,
dans lequel la lentille de contact a un coefficient de friction inférieur après un lavage pendant une nuit, comparé à une lentille témoin qui n'a pas été mise en contact avec l'agent tensio-actif contenant un alkyle aryle, mais qui est par ailleurs identique.

15. Emballage selon l'une quelconque des revendications 11 à 13 ou le procédé selon la revendication 14, dans lequel la composition polymérisable comprend du N-vinyl-N-méthylacétamide (VMA), ou de la N-vinyl pyrrolidone (NVP), ou du 1,4-butanediol vinyl éther (BVE), ou de l'éthylène glycol vinyl éther (EGVE), ou du diéthylène glycol vinyl éther (DEGVE), ou une combinaison de ceux-ci.

16. Procédé selon la revendication 14 ou la revendication 15, dans lequel la composition polymérisable comprend au moins 25 % en poids d'un monomère d'amide vinylique, ou dans lequel la composition polymérisable est exempte d'une polyvinyle pyrrolidone.

17. Procédé selon l'une quelconque des revendications 14 à 16, dans lequel, après l'étape de chauffage, la solution de conditionnement à une concentration de l'agent tensio-actif contenant du PEO qui est au moins de 20 % inférieure à la concentration de l'agent tensio-actif contenant du PEO avant la mise en contact avec le corps de lentille en polymère.
